# EUROPEAN PATENT APPLICATION

(11) **EP 4 574 058 A1**
(43) Date of publication of application: **25.06.2025**
(21) Application number: 23218100.8
(22) Date of filing: 19.12.2023
(51) Int. Cl.: A61B 10/00, A61B 10/04

(54) **INGESTIBLE DEVICE FOR SAMPLING MATERIAL USING A GAS GENERATING ACTUATOR**

(71) Applicant: Stichting IMEC Nederland, 6708 WH Wageningen (NL)
(72) Inventor: Schoeman, Rogier, 6708 RD Wageningen (NL); Elias, Jinane, 6524 PK Nijmegen (NL); de Wit, Jan Willem, 7414 HZ Deventer (NL)
(74) Representative: Patent Department IMEC

(57) **Abstract**

An ingestible device (10) for sampling material, sensing, and delivering drug at least one time is provided. The ingestible device (10) comprises a cap (12) and an electronics module (14) that are configured to slide over each other, allowing for an expansion of the ingestible device (10). Further, the electronics module (14) comprises at least one chamber having an inlet and to be filled with a material to be sampled when the inlet of the at least one chamber overlaps with an opening (32) of the cap (12), and an actuator (54) positioned at one end of the electronics module (14) covered by the cap (12). In this context, the actuator (54) is configured for, when activated, sliding of the cap (12) over the electronics module (14) so that the inlet of the at least one chamber overlaps with the opening (32) of the cap (12) to collect the material to be sampled.

## Description

The invention relates to an ingestible device for sampling material and delivering drug at least one time and a method for using such an ingestible device.

Generally, to gather information and or samples from the gastrointestinal (GI) tract, endoscopy is often performed. Endoscopes are basically long tubes that can be equipped with a camera, cutting tools, and hollow needles. The proximal and distal parts of the GI tract are reachable via endoscopy. Unfortunately, the endoscopic procedure is rather unpleasant for the patient. On top of that, the small intestine cannot be reached via this procedure. Therefore, the need for a device that enables investigation of the small intestine contents is essential. Moreover, active local drug delivery in the GI tract is challenging at the least.

US 2021/0345904 A1 relates to an ingestible capsule device which collects fluid aspirates from locations within the body, locations such as the small intestine, and retains the fluid aspirates free from contamination as the capsule device is expelled from the body. Said capsule device employs a peristaltic pump fluid control with the capsule device, and a single semi-permeable bladder store collected fluid aspirate. Disadvantageously, especially due to the usage of said peristaltic pump and the rather high energy demand and space requirement associated therewith, said ingestible capsule device cannot provide enough energy and free space for sensors exemplarily measuring GI fluid in real time. Also, due to space restriction in said ingestible capsule device, drug cannot be stored and delivered while collecting the fluid aspirates from locations within the body. In addition, there is no possibility to take multiple samples at different locations, and thereby lacking the opportunity to establish spatial concentration differences of several species present in the GI-tract. This in-turn may fail to provide insight into inflammation locations or other clinical manifestations as well as information about digestion, nutrition, and fermentation.

Accordingly, there is an object to provide an ingestible device and a method for using such an ingestible device, thereby providing enough energy and free space for sensors exemplarily measuring GI fluid in real time especially due to an energy-saving, and thus also space-saving, actuator. Also, there is an object to provide an ingestible device and method for controlled sampling material and delivering drug at one or more locations in the GI tract.

This object is solved by the features of the independent claim for an ingestible device for sampling material at least one time. The dependent claims contain further developments.

According to a first aspect of the invention, an ingestible device for sampling material in a gastrointestinal (GI) tract is provided. The ingestible device comprises a cap and an electronics module that are configured to slide over each other, allowing for an expansion of the ingestible device. The expansion of the device is referred to as an increase in the length of the device or expansion along the longitudinal axis of the device. Further, the electronics module comprises at least one chamber having an inlet and to be filled with a material to be sampled when the inlet of the at least one chamber overlaps with an opening of the cap. In addition, the electronics module comprises an actuator positioned at one end of the electronics module covered by the cap. In this context, the actuator is configured for, when activated, sliding of the cap over the electronics module so that the inlet of the at least one chamber overlaps with the opening of the cap to collect the material to be sampled. Advantageously, this allows for an energy-saving, and thus also space-saving, actuator, thereby enabling the provision of enough energy and free space for sensors exemplarily measuring GI fluid in real time.

In addition to this, it is noted that activating the actuator may especially comprise actively triggering the actuator. In one embodiment, a trigger signal may be communicated from an external device to the electronic module. Further, the received trigger signal may activate the actuator to generate gas towards the cavity in the ingestible device. The received trigger signal may activate an electrical circuit or switch in the electronics module that activates the actuator, causing the actuator to generate gas towards the cavity in the ingestible device.

With respect to the actuator, it is noted that upon using the ingestible device to collect the sample material and obtaining the device from the user, the actuator can be replaced to collect a new sample material in the GI tract.

As a further advantage, it is noted that the ingestible device can be used for applications including human patients as well as animal health.

According to a first preferred implementation form of the first aspect of the invention, the actuator comprises a gas generating cell positioned at one end of the electronics module that is covered by the cap. In this context, the gas generating cell is configured to generate gas when the actuator is activated. Also, the actuator comprises a compressed spring pushed against the gas generating cell at an end opposite to the at least one chamber. In addition, the actuator comprises a small cap having one or more holes, coupled to the compressed spring at an end opposite to the gas generating cell. In one embodiment, the gas generating cell of the actuator is coupled to the small cap to keep the gas generating cell in position and enable contact between the gas generating cell and the electronics module. This is achieved by a compressed spring or a coin cell leaf spring in between the small cap and the gas generating cell. In another embodiment, the gas generating cell of the actuator may be directly bonded to the electronics module via conductive glue, spotwelding, or wire-bonding, without using the compressed spring.

Further, the small cap is positioned in such a way that a cavity is formed between an end of the cap and the small cap. In this context, the generated gas flows through the holes of the small cap to fill up the cavity. As the gas fills up the cavity, the pressure of the gas also builds up in the cavity. As a result, mechanical force is exerted on the cap, causing the cap to slide over the electronic module. In particular, once the pressure in the cavity is above a threshold pressure, enough force is exerted on the cap to slide the cap over the electronic module. In one example, the threshold pressure may be in a range from about 0.1 to 4 bar, preferably 0.1 to 0.3 bar.

Furthermore, the cap comprises at least one hole that overlaps with the cavity to relief the pressure after full expansion of the device 10. Also, the cap may further comprise a pressure relief valve that is configured to release the gas filled in the cavity so as to prevent the pressure in the cavity from increasing above a critical pressure. In this context, the gas is released to enable depressurization of the cavity so that the ingestible device is not damaged due to extreme pressure built up in the cavity. Also, this provides safety to the patient/participant. It may be noted that the threshold pressure is less than the critical pressure. Also, it may be noted that the pressure in the cavity may be released after one or more chambers collect the material to be sampled.

According to a second preferred implementation form of the first aspect of the invention, the at least one chamber comprises a hydrophilic material that is configured to wick the material, such as GI fluid to be sampled. In this context, the hydrophilic material expands in volume when the material to be sampled is collected. In one example, the hydrophilic material may include hydrogel or a sponge like material that can absorb the material to be sampled. Also, while the hydrophilic material expands, the hydrophilic material pushes a preloaded substance out of the at least one chamber. In particular, the substance may be stored in the at least one chamber before the ingestible device is used to collect the sample material in the GI tract. The preloaded substance and the hydrophilic material are separated from one another so as to prevent contamination from one to the other. Further, when the hydrophilic material in the at least one chamber wicks the material to be sampled, the hydrophilic material expands causing the prestored or preloaded substance in the chamber to be pushed out of the ingestible device. Further advantageously, it is noted that the ingestible device can be used for simultaneously sampling the material and releasing the preloaded substance at least one time.

It is noted that the preloaded substance can exemplarily be a drug mixed with the liquid, or an inert liquid or quencher liquid, especially in the case if only sampling is desired without a medical treatment of the GI tract. In one example, the preloaded substance may comprise drugs that are used against the functional disorders of intestines.

According to a third preferred implementation form of the first aspect of the invention, the electronics module comprises at least three chambers, where the above mentioned at least one chamber is one of the at least three chambers. In this context, each chamber comprises an inlet that aligns with the opening of the cap to collect the material to be sampled when the cap slides over the electronic module. More specifically, when the cap slides over the electronic module, the opening of the cap overlaps with the inlet of one of the chambers, for example the first chamber that is closer to the opening of the cap. As a result, the material to be sampled is collected in the corresponding chamber. Since the cap has one opening, only one chamber is allowed to collect the material at a time. Hence, the other chambers are closed off by a closed surface of the cap covering the electronic module. Thereafter, when the cap slides further over the electronic module, the inlet of the other chamber, preferably the second chamber that is adjacent to the first chamber, is aligned or overlapped with the opening of the cap to collect the material to be sampled. Also, the previous chamber or the first chamber that has already collected the material is closed by the closed surface of the cap covering it. In a similar manner, all the chambers may collect the material as the cap slides over the electronics module from one end to another end of the electronic module.

Advantageously, the sliding motion/speed of the cap may be controlled by controlling the flow of gas towards the cavity. This in-turn may allow the opening of the cap to be aligned with the inlet of the chamber for a predetermined time so that sufficient sample material is collected by the respective chamber. Also, by controlling the sliding motion of the cap, the chambers may collect the sample material at one location or multiple locations in the GI tract. For example, the first chamber may collect the sample material at a first location in the GI tract. Further, when the ingestible device moves to a second location, the second chamber may collect a new sample material at this second location, while the first chamber is completely sealed off. In one embodiment, the amount of gas delivered is controlled based on a feedback signal received from one or more sensors indicating the sliding movement of the cap and the chamber exposed to the GI tract.

According to a further preferred implementation form of the first aspect of the invention, the electronics module comprises O-rings that are positioned in a first set of grooves in the electronic module, to ensure that the O-rings stay in place during the sliding motion of the cap. In one embodiment, the lipseals may be used in the place of O-rings. It may be noted that the terms "O-rings" and "lipseals" may be used interchangeably in the below description.

Further, each chamber is sealed by the O-rings and the cap covering the electronic module. More specifically, each chamber has a groove on either side of the chamber. Further, the O-rings are positioned in these grooves to seal the chamber against the surface of the cap covering the electronic module. Advantageously, these O-rings and the surface of the cap over the chamber prevents any material/liquid from flowing into the chamber, other than through the opening of the cap and the inlet of the chamber. Consequently or advantageously, the chamber and/or the sample material in the chamber is prevented from contamination.

According to another preferred implementation form of the first aspect of the invention, the electronics module comprises at least one sensor in the closed off space in between the O-rings. In this context, the sensors are configured to determine one or more parameters, preferably position of the ingestible device in the GI tract. In one embodiment, the sensors are positioned on a top surface or outer surface of the electronic module, preferably on the top surface of the chamber that is between two consecutive O-rings.

Further, the cap has an aperture that overlaps with at least one sensor when the cap slides over the surface of the electronic module. As a result, the sensor that is aligned with the aperture is exposed to the environment outside the ingestible device to measure one or more parameters in the GI tract.

In one embodiment, the sensors may be pH sensor, temperature sensor, pressure sensor, acceleration sensor, Oxidation Reduction Potential (ORP) sensor. Further, the parameters may include pH value, temperature, pressure, acceleration, ORP value, or any other biological analytes. In addition, the device may include these sensors at the uncovered end of the electronics module so that the sensors are always exposed to the GI tract to provide the location information of the device. Advantageously, this information may aid in determining position of the ingestible device in the GI tract that in-turn enables the ingestible device to sample the material at the correct location in the GI tract. Also, the position of the ingestible device may aid in activating the actuator at a desired location in the GI tract. This in-turn causes the chambers to collect the material at one location or multiple locations in the GI tract.

In another embodiment, the electronics module comprises an additional sensor that is outside these chambers. Further when the ingestible device is in an inactivated state, the additional sensor is aligned with an aperture of the cap so that the additional sensor is continuously exposed to the environment or GI fluid that is outside the ingestible device. Also, the additional sensor measures parameters that aid in determining position of the ingestible device in the GI tract, which in-turn enables to activate the actuator of the ingestible device. Once the actuator is activated, the cap slides over the electronics module and one or more chambers collect the sample material at a precise or desired location in the GI tract.

According to another preferred implementation form of the first aspect of the invention, the cap comprises one or more ridges that fit in a second set of grooves in the electronic module. In this context, the ridges are provided on the inner surface of the cap. On the other hand, the second set of grooves are provided on the outer surface of the electronic module. Further, each ridge is aligned to a corresponding groove. Also, these ridges lock with the second set of grooves in the electronics module so that the cap is prevented from rotating over the electronic module. Particularly, the rotation of the cap may misalign the position of the opening of the cap with respect to the inlet of the chamber. Consequently, the chamber may fail to collect the material when the cap slides over the electronic module. Thus, it is important to prevent the rotation of the cap over the electronic module.

According to a further preferred implementation form of the first aspect of the invention, the material comprises or is gastrointestinal content or fluid in the GI tract. Additionally or alternatively, the preloaded substance comprises or is a drug. Advantageously, for example, a simultaneous medical treatment of the GI tract is enabled in a particularly efficient manner.

According to a further preferred implementation form of the first aspect of the invention, at least a part of the inner space of each chamber comprises a stabilizing substance, preferably a quencher, especially for preventing the material from additional chemical reactions. In one example, the quencher may be embedded in the wicking material. Advantageously, for instance, digestion and fermentation of the sampled content is prevented in a particularly efficient manner.

According to a further preferred implementation form of the first aspect of the invention, the ingestible device further comprises a body portion especially providing a hollow inner space being preferably sealed against the environment of the body portion. In this context, the electronics module may have a hollow inner space adjacent to the chambers. This hollow inner space may be used as the body portion for holding or positioning one or more electronics in the ingestible device.

According to a further preferred implementation form of the first aspect of the invention, the body portion, especially the hollow inner space of the body portion, comprises at least one of an energy source for supplying electric power to the sensors and other electronic modules. In one embodiment, the actuator may need power from the energy source of the electronics module to switch ON the gas generating cell. Further, when the actuator receives the trigger signal, the gas generator or the gas generating cell is activated to generate gas towards the small cap. Further, the body portion comprises a trigger unit, being especially supplied the electric power by the energy source, and configured to receive at least one trigger signal in order to trigger or activate the actuator. In addition, the body portion comprises a data recording unit for recording data, preferably environment data and/or localization data, during usage of the ingestible device.

Furthermore, the body portion comprises a wireless data transmission unit for wirelessly transmitting data, preferably environment data and/or localization data, more preferably environment data and/or localization data in real time, during usage of the ingestible device. Additionally or alternatively, the body portion, especially not the hollow inner space of the body portion, comprises at least one sensor, preferably a pH-value sensor. Advantageously, for instance, there is plenty of room for the energy source, electronics such as communication, preferably wireless communication, and at least one antenna, and sensors.

According to a further preferred implementation form of the first aspect of the invention, the ingestible device has the shape of a pill or a cylinder. Additionally or alternatively, the length of the ingestible device is 31 millimeters after the actuator has been triggered or activated. Further additionally or further alternatively, the diameter of the ingestible device is lower than 10 millimeters, especially lower than 9.9 millimeters, preferably after the actuator is activated. Advantageously, for example, the ingestible device is small enough in size that it can easily be swallowed.

According to a second aspect of the invention, a method for using an ingestible device according to any of the implementation forms of the first aspect of the invention is provided. The method comprises the step of triggering or activating the actuator by a trigger signal. In this context, after activation, the gas generating cell in the actuator generates the gas that fills the cavity between the cap and the small cap of the electronic module. Once the pressure of the gas is above the threshold pressure, the cap slides over the electronics module so that the inlet of the chamber overlaps with the opening of the cap to collect the material to be sampled.

Exemplary embodiments of the invention are now further explained with respect to the drawings by way of example only, and not for limitation. In the drawings:
- Fig. 1: shows an exemplary embodiment of an inventive ingestible device in an inactivated state;
- Fig. 2: shows the exemplary embodiment according to Fig. 1, respectively, as a blow-up drawing;
- Figs. 3a-3i: shows a time lapse of the ingestible device from an inactivate state of FIG. 1 to an activate state;
- Fig. 4a-4b: shows the illustration of ridges of a cap fit into grooves of an electronics module of the ingestible device according to Fig. 1;
- Fig. 5: shows a block diagram of a body portion, an actuator, and an external device, in accordance with the aspects of the invention.

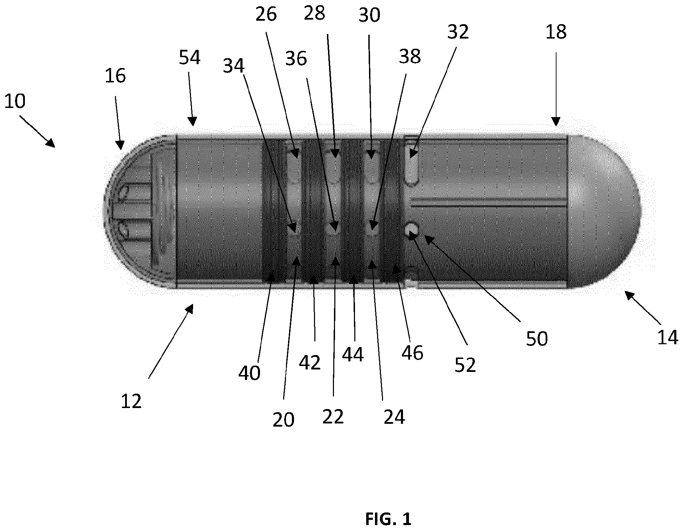

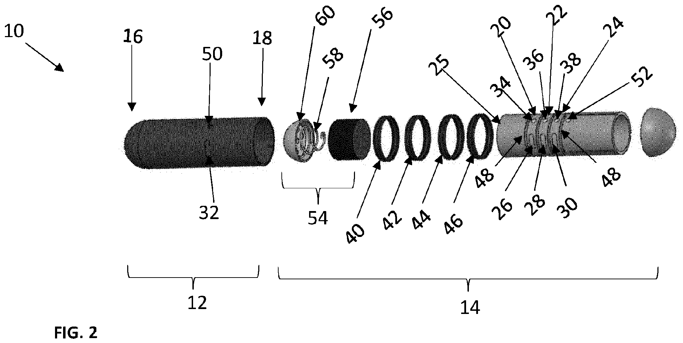

With respect to Figs. 1 and 2, an exemplary embodiment of an ingestible device 10 for sampling material and optionally, releasing substance or drug at least one time is illustrated. Fig. 1 depicts the ingestible device 10 in an inactivated state. Fig. 2 depicts a blow-up view of the ingestible device 10. It may be noted that the inactivated state may be referred to as a state where the actuator is not triggered or activated to release gas into the ingestible device 10.

According to Fig. 1, the ingestible device 10 comprises a cap 12 and an electronics module 14 that are configured to slide over each other, allowing for an expansion of the ingestible device 10. The cap 12 may be referred to as a tubular structure with the first end 16 closed while the second end 18 is open. Further, during the inactivated state, a substantial portion of the electronics module 14 is positioned inside the tubular structure of the cap 12 as depicted in Fig. 1. In one embodiment, the electronics module 14 may be inserted or pushed into the cap 12 via the second end 18 of the cap 12. It may be noted that, in Fig. 1, the cap 12 is shown translucent so that the components of the electronics module 14 are visible when the electronic 14 module is positioned inside the cap 12.

Further, the cap 12 and the electronics module 14 are assembled in such a way that they slide over each other. In particular, the cap 12 slides over the electronic module, causing the expansion of the ingestible device 10. It may be noted that the cap 12 is intact with the electronics module 14 while the cap 12 slides over the electronics module 14. Also, the cap 12 and the electronics module 14 are assembled in such a way that they are not separated from each other even after complete activation or usage of the ingestible device 10 to collect the sample material. Advantageously, this allows the cap 12 and the electronics module 14 to stay together as one component in the GI tract.

In accordance with Figs. 1 and 2, the electronics module 14 comprises at least one chamber having an inlet and to be filled with a material to be sampled when the inlet of the at least one chamber overlaps with an opening of the cap. It is noted that the chamber takes in the material through the inlet especially due to an under pressure. As depicted in Figs. 1 and 2, the electronics module 14 comprises three chambers 20, 22, 24 that are adjacent to one another along the length of the electronics module 14. These chambers may also be referred to as a first chamber 24, a second chamber 22, and a third chamber 20 in the below description. It may be noted that the ingestible device 10 may comprise any number of chambers and is not limited to three chambers as shown in Figs. 1 and 2. Further, each of the chambers 20-24 comprises an inlet 26-30 that allows the material to flow into the chamber when an opening 32 of the cap 12 overlaps with the inlet 26-30 of the corresponding chamber 20-24. In one example, the material may be referred to as gastrointestinal (GI) content or fluid that is collected when the ingestible device 10 passes through the GI tract of a body such as, human body or animal body. It may be noted that the chamber may be referred to as a compartment that is capable of storing the material.

Furthermore, one or more chambers 20-24 comprises a hydrophilic material that is configured to wick the material to be sampled. In one example, the hydrophilic material wicks the intestinal fluid into the corresponding chamber 20-24. In this context, the hydrophilic material expands in volume when the material to be sampled is collected. In one example, the hydrophilic material may include hydrogel or a sponge like material that can absorb the material to be sampled.

In addition, while the hydrophilic material expands, the hydrophilic material pushes a preloaded substance out of the chamber 20-24. It is noted that each of the chambers 20-24 takes in the material through their respective inlet 26-30 especially due to under pressure and releases the preloaded substance especially due to an over pressure. In particular, the substance may be stored in the chambers 20-24 before the ingestible device 10 is used to collect the sample material in the GI tract. Further, when the hydrophilic material in the chambers 20-24 wick the material to be sampled, the hydrophilic material expands causing the prestored or preloaded substance in the chambers 20-24 to be pushed out of the ingestible device 10. Further advantageously, it is noted that the ingestible device 10 can be used for simultaneously sampling the material and releasing the preloaded substance at least one time.

In one example, the preloaded substance may be referred to as one or more drugs either in a liquid state or mixed with other known liquids to be released or delivered within the GI tract when the ingestible device 10 passes through the GI tract of the body. In one example, the drugs may be used against the functional disorders of intestines.

Furthermore, the chambers 20-24 are separated by O-rings 40, 42, 44, 46 that are positioned on the grooves 48 of the electronics module 14. These grooves 48 may be referred to as a first set of grooves. More specifically, each chamber 20-24 has grooves 48 on either side of the chamber as depicted in Figs. 1 and 2. Also, these grooves 48 are circular grooves of a predetermined width. Further, the O-rings 40-46 are placed in these grooves 48, to ensure they stay in place during the sliding motion of the cap 12. Also, these O-rings 40-46 may act as a barrier on the top surface of the chambers 20-24 to seal the chambers 20-24. More specifically, the O-rings 40-46 on either side of the chamber and the cap 12 on the top surface of the chamber may completely seal the chamber and keep the chamber watertight. The material may flow into each chamber 20-24 only when the respective inlet of the chamber overlaps with the opening 32 of the cap 12. Otherwise, the chamber is tightly sealed to prevent any contamination of the chamber and/or material in the chamber.

In accordance with Figs. 1 and 2, the electronics module 14 comprises multiple rows of sensors 34, 36, 38. In particular, the electronics module 14 comprises one or more sensors 34-38 that are positioned in the closed off space in between the lipseals or O-rings 40-46 as depicted in Figs. 1 and 2. The closed off space may be referred to as the outer surface area of the electronics module 14 between the O-rings 40-46. This outer surface area may also be the closed surface of one of the chambers 20-24. This positioning enables clean sensors 34-38 to take measurements in the GI tract. This feature is especially important because the sensors 34-38 are continuously exposed to the harsh environment of the GI tract where sensor surface fouling is a problem. Also, these sensors may get damaged due to fouling. Hence, by positioning the sensors 34-38 in the closed off space in between the O-rings 40-46, the sensors 34-38 are protected from surface fouling. Also, some sensors may be one-time use so it is advantageous to cover these sensors 34-38 and expose them one after another to measure at multiple positions/locations in the GI tract. Moreover, because the aperture 50 in the sliding cap 12 overlaps with a new sensor each step, the pill or device 10 can keep on measuring for a longer period of time. Also, the sensors 34-38 are sealed off from the outside until the aperture 50 aligns with the respective sensor to expose it to the environment and may function as a new fresh sensor.

Furthermore, these sensors 34-38 are used to determine one or more parameters in the GI tract. In one example, the parameters may include one or more values of the biological analytes. In this context, the cap 12 has an aperture 50 that overlaps with at least one sensor 34-38 when the cap 12 slides over the surface of the electronics module 14. As a result, the sensor that is aligned with the aperture 50 is exposed to the environment outside the ingestible device 10 to measure one or more parameters in the GI tract.

Further, these determined parameters may be used to determine the location of the ingestible device 10 in the GI tract. Also, the sensors 34-38 may be one or more types to measure biological analytes. Moreover, determination of the position/location of the device 10 is crucial for interpreting samples and in vivo measurements. In one embodiment, the electronics module 14 comprises sensors 34-38 that aid in continuous measurements in the GI-tract.

In accordance with the embodiment of Figs. 1 and 2, the electronics module 14 comprises an additional sensor 52 that is outside these chambers 20-24. Further when the ingestible device 10 is in an inactivated state, this additional sensor 52 is aligned with the aperture 50 of the cap 12 so that the additional sensor 52 is continuously exposed to the environment or GI fluid that is outside the ingestible device 10, as depicted in Fig. 1. Also, the additional sensor 52 measures parameters that aid in determining the position/location of the ingestible device 10 in the GI tract, which in-turn enables to activate the ingestible device 10 so that the first sampling of the material takes place at a precise or desired location in the GI tract. Further when the ingestible device 10 is in inactivated state, the other rows of sensors 34-38 are positioned in the closed off compartments, allowing them to remain clean until the respective chamber 20-24 opens or the ingestible device 10 is switched to an activated state. As mentioned before, this feature is especially important otherwise, the sensors 34-38 may be continuously exposed to the harsh environment of the GI tract where sensor surface fouling is a problem.

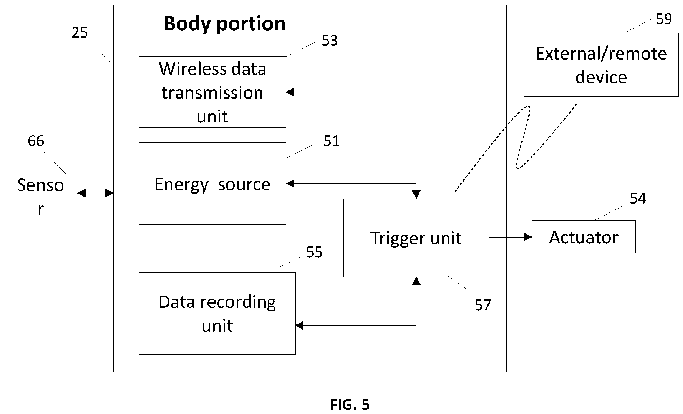

In addition to the chambers 20-24, the electronics module 14 comprises an actuator 54 (see Fig. 2) positioned at one end of the electronics module 14. In this context, the actuator 54 is configured such that activating the actuator 54 leads to sliding of the cap 12 over the electronics module 14 so that the inlet of the at least one chamber overlaps with the opening of the cap to collect the material to be sampled. The aspect of activating the actuator 54 is described in greater detail with respect to Fig. 5. As depicted in Figs. 1 and 2, the actuator 54 comprises a gas generating cell 56 positioned at one end of the electronics module 14 that is covered by the cap 12. In this context, the gas generating cell 56 is configured to generate gas when the actuator 54 is activated. In one example, the gas generating cell 56 may be a hydrogen gas generating cell that is placed lateral in the electronics module 14, and configured to generate hydrogen gas or any other similar gas that may help to slide the cap 12 over the electronics module 14.

Furthermore, the actuator 54 comprises a compressed spring 58 (see Fig. 2) pushed against the gas generating cell 56 at an end opposite to the chambers 20-24. In this context, the compressed spring 58 may be a conical compression spring that is used to compress the gas generating cell 56.

In addition, the actuator 54 comprises a small cap 60 that is coupled to the compressed spring 58 at an end opposite to the gas generating cell 56. In this context, the small cap 60 is attached to the compressed spring 58 to keep the gas generating cell 56 at a desired position in the electronics module 14. In one embodiment, the gas generating cell 56 of the actuator 54 is coupled to the small cap 60 to keep the gas generating cell 56 in position and enable contact between the gas generating cell 56 and the electronics module 14. This can be achieved by a compressed spring or a coin cell leaf spring between the small cap 60 and the gas generating cell 56. More specifically, the compressed spring pushes the gas generating cell 56 against the electrical surface of the electronics module 14 so that there is contact between the gas generating cell 56 and the electronics module 14. Further, when a switch or electrical circuit in the electronics module is turned ON or closed, the gas generating cell 56 is activated to generate gas towards the small cap 60.

In another embodiment, the gas generating cell 56 of the actuator 54 may be directly bonded to the electronics module 14 via conductive glue, spotwelding, or wire-bonding, without using the compressed spring 58. It may be noted that the small cap 60 and the compressed spring 58 are optional elements in the actuator 54. There may be other means to position the gas generating cell 56 in the actuator 54.

Also, the small cap 60 includes one or more holes to allow the gas to pass through. Moreover, the small cap 60 is positioned in such a way that a cavity is formed between the end 16 of the cap 12 and the small cap 60. In particular, when the electronics module 14 is placed inside the cap 12, the small cap 60 is positioned close to the first end 16 of the cap 12. Also, a gap is formed between the small cap 60 and the first end 16 of the cap 12. This gap is referred to as the cavity in the ingestible device 10.

Further when the actuator 54 is activated, the gas generating cell 56 generates gas that flows through the holes of the small cap 60 to fill up the cavity. In this context, the gas flow into the cavity may lead to pressure build up in the cavity, causing a sliding motion of the cap 12. In particular, due to the gas pressure built up in the cavity, a mechanical force is exerted on the cap 12 to push the cap 12 away from the electronics module 14. As a consequence, the cap 12 slides over the electronics module 14. In one example, the gas includes hydrogen gas. In particular, the cap 12 slides over the electronics module 14 when the pressure built up in the cavity is above a threshold pressure. The threshold pressure is in a range from about 0.1 to 4 bar, preferably 0.1 to 0.3 bar.

Additionally, the cap 12 comprises at least one hole that overlaps with the cavity to release the gas filled in the cavity so as to prevent the pressure in the cavity from increasing above a critical pressure. In this context, the cap 12 includes one or more holes at a location that is far from the cavity when the ingestible device 10 is in an inactivated state. Further, as the cap 12 slides over the electronics module 14 due to pressure built up, the cavity also enlarges in space. Once the chambers 20-24 are filled with material to be sampled, the gas generating cell 56 stops to generate further gas. Even if the gas generating cell 56 generates further gas, the cavity may overlap with the one of more holes in the cap 12, causing to release the gas filled in the cavity. This in-turn prevents the pressure in the cavity from increasing above a critical pressure.

Additionally, the cap 12 may further comprise a pressure relief valve that is configured to release the gas filled in the cavity so as to prevent the pressure in the cavity from increasing above a critical pressure. It is important to have the pressure relief valve because at times, the cavity may not overlap with the holes in the cap 12. For example, the cap 12 may not slide up anymore due to fouling. In these situations, the pressure relief valve helps to release the gas and prevents the pressure build up reaching the critical pressure value. The gas is released to enable depressurization of the cavity so that the ingestible device is not damaged due to extreme pressure built up in the cavity. This in-turn provides safety to the patient/participant using the device 10.

In accordance with Figs. 1 and 2, at least a part of the inner space of the at least one chamber comprises a stabilizing substance, preferably a quencher, especially for preventing the material from additional chemical reactions. In this context, the chambers 20-24 of the electronics module 12 are provided with the quencher that aids in stabilizing the material that is sampled. In particular, the ingestible device 10 may remain quite some time, e.g., 72 hours, in the body after sampling. To prevent the sample material from deteriorating in this time, the sample material is stabilized by a quencher, i.e., a chemical compound preventing further chemical reaction such as digestion and fermentation of the sampled content. In one embodiment, the quencher may be embedded in the wicking material/hydrogel in the chamber. In another embodiment, the chamber may be without the stabilizing substance, particularly when sampling is not required but only the sensing of the parameters in the GI tract and/or drug delivery is required or performed.

Furthermore, as can be seen from Figs. 1 and 2, once the ingestible device 10 is out of the body, the ingestible device 10 is retrieved, and the sample material is easily removed from the chambers 20-24. More specifically, after the ingestible device 10 is retrieved, the cap 12 can be slid off and the hydrophilic material containing the sample material is removed from the sampling chambers of the electronics module 14. Further, the sample material is retrieved from the removed hydrophilic material.

In accordance with Fig. 1, the electronics module 14 further comprises a body portion 25 especially providing a hollow inner space being preferably sealed against the environment of the body portion 25. In this context, the body portion may be the hollow inner space of the electronics module that is between the gas generating cell and the chambers.

It might be particularly advantageous if the body portion 25, especially the hollow inner space of the body portion 25, comprises at least one of an energy source 51 (see Fig. 5) for supplying electric power to the electronics module 14. The electronics module 14 is large enough to contain the energy source 51 that is powerful enough to supply sufficient energy to the sensors 34, 36, 38, 52, the actuator 54, and other modules e.g., communication modules in the device 10.

Furthermore, the body portion 25 comprises a trigger unit 57 (see Fig. 5), being especially supplied the electric power by the energy source 51, and configured to receive at least one trigger signal in order to trigger or activate the actuator 54. In one example, the trigger signal may be a radio frequency (RF) communication signal received from an external device 59 (see Fig. 5).

Further, the body portion 25 comprises a data recording unit 55 (see Fig. 5) for recording data, preferably environment data and/or localization data, during usage of the ingestible device 10. In one example, the environment data could be pH and temperature. This data is also used to roughly determine the position of the pill in the GI tract. Other environmental data could be inflammation markers or other biological analytes. It may be noted that the body portion 25 is described in greater detail with respect to Fig. 5.

In addition to this or as an alternative, the body portion 25, especially not the hollow inner space of the body portion 25, may comprise one or more sensors 34-38. In one example, a pH-value sensor may be used to determine or measure the pH-value at one or more locations in the GI tract. It is further noted that the ingestible device 10 has the shape of a pill or a cylinder.

As it can be seen from Fig. 1 showing the inactivated state of the ingestible device 10, the diameter of the ingestible device 10 does not change after activation, whereas the length of the ingestible device 10 increases due to activation. In this context, the length of the ingestible device 10 is lower than 31 millimeters, exemplarily after the actuator is activated. Also, the diameter of the ingestible device 10 is lower than 9.9 millimeters.

With respect to the inactivated state of the ingestible device 10 according to Fig. 1, it is noted that in this state, the actuator 54 has not been triggered yet. Accordingly, the cap 12 substantially encloses the electronics module 14 and the opening 32 of the cap 12 is close to the end of the electronics module 14 that is far away from the small cap 60, as depicted in Fig. 1. With respect to the activated state of the ingestible device 10, it is further noted that in this state, the actuator 54 has been triggered or activated, which leads to the cap 12 sliding over the electronics module 14, allowing for the expansion of the ingestible device 10. Also, the chambers 20-24 in the electronics module 14 collect the material to be sampled.

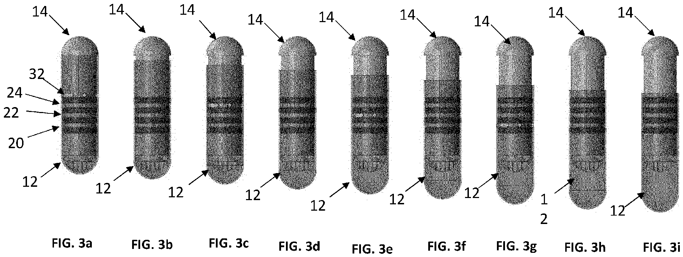

Referring to Figs. 3a-3i, a time lapse of the ingestible device 10 from an inactivated state of FIG. 1 to an activated state, in accordance with the aspects of the invention is depicted. In Fig. 3a, the ingestible device 10 is in an inactivated state. Hence, the cap 12 and the electronics module 14 are substantially overlapping one another. Also, the small cap 60 of the electronics module 14 is close to the first end 16 of the cap 12. Further, when the actuator 54 is activated, the gas generating cell 56 generates gas towards the cavity. When the pressure of the gas in the cavity is above the threshold pressure, the cap 12 begins to slide over the electronics module 14, as depicted in Fig. 3b. Also, as the cap 12 begins to slide, the opening 32 of the cap 12 is under the first O-ring 46, and it is not visible in Fig. 3b.

Further, Fig. 3c shows that the opening 32 of the cap 12 overlaps with the inlet 30 of the first chamber 24 due to sliding motion of the cap 12 over the electronics module 14. In this context, the material such as the intestinal fluid in the GI tract may flow into the first chamber 24. Also, the hydrophilic material in the first chamber 24 wicks the material and stores it in the first chamber 24. Moreover, while the first chamber 24 is collecting the material to be sampled, the other chambers, such as the second and third chambers 20, 22 are covered or sealed by the closed surface of the cap 12 and the O-rings 40-46 on either side of each chamber 20, 22, as depicted in Fig. 3c.

Additionally, Fig. 3d shows that the opening 32 of the cap 12 overlaps with the second O-ring 44 that is adjacent to the first chamber 24, due to further sliding motion of the cap 12. At this stage, all three chambers 20-24 are sealed, and the opening 32 of the cap 12 overlaps with the second O-ring 44. Thereafter, when the cap 12 slides further, the opening 32 of the cap 12 also moves from the second O-ring 44 towards the inlet 28 of the second chamber 22, as depicted in Fig. 3e. Once the opening 32 of the cap 12 completely overlaps with the inlet 28 of the second chamber 22, the material flows or is wicked by the hydrophilic material in the second chamber 22.

Fig. 3f shows that the cap 12 slides further and thereby, the opening 32 of the cap 12 also moves from the inlet 28 of the second chamber 22 to the third O-ring 42. Furthermore, in Fig. 3g. the opening 32 of the cap 12 overlaps with the inlet 26 of the third chamber 20 to fill the third chamber 20 with the material to be sampled. Thereafter, Fig. 3h shows that the opening 32 of the cap 12 has moved from the inlet 26 of the third chamber 20 towards the fourth O-ring 40. In this context, the opening 32 of the cap 12 is below the fourth O-ring 40. Also, all three chambers 20-24 are sealed so that the materials in these chambers 20-24 are not contaminated. Finally, Fig. 3i shows that the cap 12 slides further and the opening 32 of the cap 12 crosses the fourth O-ring 40. At this stage, the chambers 20-24 have collected the material. Also, the gas generating cell 56 stops producing/generating gas. Even if there is further generation of gas by the gas generating cell 56, the cap 12 slides further so that the opening 32 of the cap 12 overlaps with the cavity. Consequently, the gas in the cavity escapes through the opening 32 of the cap 12. Thus, the pressure is relieved after full expansion of the device 10.

Additionally or alternatively, the cap 12 may further comprise a pressure relief valve that is configured to release the gas filled in the cavity so as to prevent the pressure in the cavity from increasing above a critical pressure.

In another embodiment, the cap 12 may include one or more holes on the side that is opposite to the opening 32 of the cap 12. Also, these holes are placed in such a way that once the chambers 20-24 collect the sampled material, the holes align with the cavity to release the gas in the cavity. In this way, the gas is released through the holes much sooner than through the opening 32 of the cap 12.

In one embodiment, the overall sliding motion/speed of the cap 12 over the electronics module 14 may be controlled based on the gas flow from the gas generating cell 56. This in-turn helps the ingestible device 10 to collect the sample material at one or more locations in the GI tract. For example, the gas generating cell 56 is triggered or activated by a first trigger signal so that the cap 12 slides over a first distance. Here, the first chamber 24 collects the sample material at a first location in the GI tract. Further, when the ingestible device 10 moves to a second location in the GI tract, the gas generating cell 56 can be triggered or activated by a second trigger signal so that the cap 12 slides further to a second distance. Here, the second chamber 22 collects a new sample material at this second location, while the first chamber 24 is completely sealed off. In a similar manner, the third chamber 20 collects the sample material at a third location in the GI tract. In one embodiment, after receiving a trigger signal, the cap 12 may continuously slide over each of the chambers, causing the respective chambers to open-up to collect the material to be sampled. Here, only one trigger signal is enough to perform the sampling at each of the chambers. The option of sampling at multiple locations may prevent not only the patient from having to take several pills, but also provides the opportunity to establish spatial concentration differences of several species present in the GI-tract.

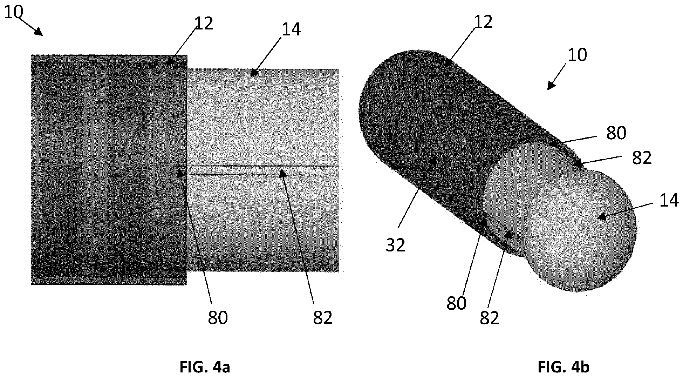

Figs. 4a-4b illustrates ridges of a cap fit into grooves of an electronics module of the ingestible device 10, according to the aspects of the invention. The cap 12 comprises one or more ridges 80 that fit in a second set of grooves 82 in the electronics module 14. In this context, the ridges 80 are provided on the inner surface of the cap 12. On the other hand, the second set of grooves 82 are provided on the outer surface of the electronics module 14. Further, each ridge 80 is aligned to a corresponding groove 82. Also, these ridges 80 lock with the second set of grooves 82 in the electronics module 14 so that the cap 12 is prevented from rotating over the electronics module 14. Particularly, the rotation of the cap 12 may misalign the position of the opening 32 of the cap 12 with respect to the inlet of the chambers 20-24. Consequently, the chambers 20-24 may fail to collect the material when the cap 12 slides over the electronics module 14. Thus, it is important to lock the ridges 80 with the grooves 82 so as to prevent the rotation of the cap 12 over the electronics module 14.

Referring to Fig. 5, a block diagram of a body portion 25, an actuator 54, and an external device 59, in accordance with the aspects of the invention, is depicted. With respect to the body portion 25, it is noted that said body portion, especially its hollow inner space, may comprise one or more modules as depicted in Fig. 5. It might be particularly advantageous if said body portion comprises at least an energy source 51, a trigger unit 57, a data recording unit 55, and a wireless data transmission unit 53. The energy source 51 is configured to supply electric power to the actuator 54. Also, the energy source 51 may supply electric power to the trigger unit 57, the data recording unit 55, and the wireless data transmission unit 53. Further, the trigger unit 57 is configured to receive at least one trigger signal in order to trigger or activate the actuator 54. In one example, the trigger unit 57 may receive the trigger signal from an external or remote device 59 as shown in Fig. 5. In one example, the trigger signal may be a RF communication signal.

In one embodiment, the trigger unit 57 includes a controller or a microcontroller to steer the activation of the actuator 54 and also to store the data such as environment data and/or localization data in the data recording unit 57. The controller may steer the activation of the actuator 54 either based on the receipt of the trigger signal from the external device 59 or based on the sensor readings within the device. For the latter part, in one example, the controller in the trigger unit 57 may receive one or more parameters e.g., pH value from the sensor 66 that indicates the location of the device 10 in the GI tract. Further, once the device 10 has reached a desired location in the GI tract, the controller in the trigger unit 57 may send a trigger signal to the actuator 54 to activate the actuator 54.

In another embodiment, a timer may be coupled to the controller and the timer may send a signal to the controller to activate the actuator 54. The timer may send a signal after a predetermined time set by the user or based on detecting the entry of the device 10 to a particular part e.g., the duodenum, of the GI tract by means of the PH change sensed by the pH sensor 66 in the device 10. In one example, a PH sensor 66 is used to detect that the pill or device 10 is going into the smaller intestine and the controller in the trigger unit 57 may start a timer so that the actuator 54 is activated every 20 minutes to collect the sample from the GI tract.

In this context, if the actuator 54 is triggered or activated, the energy source 51 is coupled to the actuator 54. Once the power or energy source 51 is coupled to the actuator 54, the gas generated cell 56 is activated to generate the gas towards the cavity in the ingestible device 10. As a consequence, the cap 12 slides over the electronics module 14, and the chambers 20-24 collect the material to be sampled. In another embodiment, the actuator, such as the hydrogen actuator does not need an external power source, because the reaction takes place within the hydrogen actuator. In this context, the trigger unit 57 may directly trigger or activate the actuator to generate the gas towards the cavity.

Furthermore, the data recording unit 55 is configured to record data, preferably environment data and/or localization data, during usage of the ingestible device 10. The wireless data transmission unit 53 is configured to wirelessly transmit data, preferably environment data and/or localization data, more preferably environment data and/or localization data in real time, during usage of the ingestible device 10. In one embodiment, the wireless data transmission unit 53 may also be configured to wirelessly receive the trigger signal from the external device 59 and transmit the trigger signal to the trigger unit 57. In addition, the said electronics module may comprise at least one sensor 66, preferably a pH-value sensor for sensing pH-value at a desired location in the GI tract.

The exemplary embodiment of the inventive ingestible device 10 described above can be seen as a remotely activatable sampling, sensing, and drug delivery pill, which especially consists of only a few elements. Thus, manufacturing such an ingestible device is advantageously very cheap.

The cap 12 and the electronics module 14 are strong enough to withstand some pressure. Therefore, it can function as a strut to keep the outer shell of the ingestible device 10 fixed in the inactivated state. Also, the chambers 20-24 can be filled or preloaded with a substance or drug containing liquid resulting a dual functionality for controlled sampling actuation and drug delivery. In addition, the ingestible device contains fewer complex mechanical parts and a simpler design compared to US 2021/0345904 A1. Therefore, costs of manufacturing and assembly can be much lower. Also, the reliability of the actuator can be much better.

Advantageously, the sliding motion of cap 12 enables easy access to the sample chamber 20-24. This can be very helpful for effortless injecting a quencher as well as simple sample removal. The overall size of the remotely activatable sampling pill in the inactivated state is not exceeding 26.9 millimeters in length and/or 9.9 millimeters in diameter, which advantageously makes the pill relatively easy to swallow. Advantageously, the hollow electronics module or the hollow body portion, respectively, has enough space inside to implement the power/energy source, the sensors, and further electronics such as communication, preferably wireless communication, and at least one antenna.

Moreover, as a further advantage, the sensors are implemented in the closed off space in between the O-rings to protect the sensors from surface fouling. Also, these sensors are timely exposed to the gastrointestinal (GI) fluid. Accordingly, sensors implemented in this area of the pill can therefore measure GI fluid in real time. These measurements contribute the place determination of the pill and give insight in patient health or animal health, respectively.

It is particularly advantageous that this actuator requires very little voltage, and therefore avoids having to make use of large batteries making it extremely suitable for the remotely activatable sampling pill.

As already discussed above, the inventive ingestible device advantageously provides closed off compartments or chambers because the sample chambers are sealed off by lipseals or O-rings and the closed surface of the cap. Further advantageously, both components are very cheap and easily obtainable. As a further advantage, the inventive ingestible device allows for easy sample removability. Accordingly, the chance of sample spillage is made non-existent.

Furthermore, as already discussed above, the ingestible device according to the invention provides possible space for sensors because the electronics module of the remotely activatable sampling pill or the ingestible device is large enough to contain sensors. The sensors can advantageously determine position to enable sampling at the correct location in the GI tract. Moreover, this also allows for continuous measurements of GI fluid.

The major advantages of the remotely activatable dual-sampling pill or the inventive ingestible device, respectively, can be summarized in a nutshell as follows:
1. The device allows for noninvasive active gastrointestinal fluid sampling.
2. The device is simple to fabricate exemplarily via 3D printing. Other parts are easily obtainable.
3. The sliding aspect of the cap enables effortless sample retrieval. Also, the sliding mechanism is an elegant way to achieve sampling, while diminishing the chance of failure.
4. The spacious interior of the electronics module leaves plenty of room for the energy source, electronics, and sensors. The implemented sensors that measure GI fluid in real time contribute to the place determination of the pill, and give insight in patient health. These sensors are continuously exposed to the environment to allow for longer measurements.
5. Further, the sensors above each of the chambers are covered by the cap and are exposed one after the other so that fouling is mitigated. Also, this enables the use of one-time sensors.
6. Due to overall pill size, in the inactivated state, not exceeding the already discussed size, the administration of the pill is comfortable for the user.
7. The remotely activatable sampling pill in its entirety is nonmagnetic, making it MRI compatible.
8. The remotely activatable sampling pill comprises a build in localization component.
9. There is no sample contamination due to closed-off or sealed sample chambers by the O-rings and the cap.
10. The remotely activatable sampling pill is very easy to assemble.
11. Due to overall pill size, in the activated state, not exceeding the size as discussed above, the chance of pill retention is strongly reduced.
12. In addition to sampling, the remotely activatable sampling pill can deliver drug into the GI tract (for instance, drug delivery). The same actuation mechanism is used for sampling and simultaneous delivery.
13. The possibility to take multiple samples also allows sampling at different locations. This option prevents not only the patient from having to take several pills, but also provides the opportunity to establish spatial concentration differences of several species present in the GI-tract. This in-turn may give insight into inflammation locations or other clinical manifestations as well as information about digestion, nutrition, and fermentation.
14. In addition, the fact that both a sensor and a sampling chamber can be exposed simultaneously, allows for sensor validation after retrieval of the device. This can be done through a lab analysis performed on the retrieved sample. Thus, this device can be a tool to validate the accuracy of miniature sensors, against standard laboratory equipment.

While various embodiments of the present invention have been described above, it should be understood that they have been presented by way of example only, and not limitation. Numerous changes to the disclosed embodiments can be made in accordance with the disclosure herein without departing from the spirit or scope of the invention. Thus, the breadth and scope of the present invention should not be limited by any of the above described embodiments. Rather, the scope of the invention should be defined in accordance with the following claims and their equivalents.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. An ingestible device (10) for sampling material in a gastrointestinal (GI) tract, the ingestible device (10) comprising:
a cap (12) and an electronics module (14) that are configured to slide over each other, allowing for an expansion of the ingestible device (10),
wherein the electronics module (14) comprises:
at least one chamber having an inlet and to be filled with a material to be sampled when the inlet of the at least one chamber overlaps with an opening (32) of the cap (12); and
an actuator (54) positioned at one end of the electronics module (14) covered by the cap (12), wherein the actuator (54) is configured for, when activated, sliding of the cap (12) over the electronics module (14) so that the inlet of the at least one chamber overlaps with the opening (32) of the cap (12) to collect the material to be sampled.

2. The ingestible device (10) according to claim 1, wherein the actuator (54) comprises:
a gas generating cell (56) positioned at one end of the electronics module (14) that is covered by the cap (12), wherein the gas generating cell (56) is configured to generate gas when the actuator (54) is activated; and
a small cap (60) having one or more holes, coupled to the gas generating cell (56).

3. The ingestible device (10) according to claim 2, wherein the generated gas flows through the holes of the small cap and fills up a cavity formed between an end of the cap (12) and the small cap (60), leading to pressure build up in the cavity so that a mechanical force is exerted on the cap (12) to slide over the electronics module (14).

4. The ingestible device (10) according to claim 3, wherein the cap (12) slides over the electronics module (14) when the pressure built up in the cavity is above a threshold pressure.

5. The ingestible device (10) according to claim 3, wherein the cap (12) comprises at least one hole that overlaps with the cavity to release the gas filled in the cavity so as to prevent the pressure in the cavity from increasing above a critical pressure.

6. The ingestible device (10) according to any of the preceding claims, wherein the at least one chamber comprises a hydrophilic material having hydrogel that is configured to wick the material to be sampled.

7. The ingestible device (10) according to claim 6, wherein the hydrophilic material expands in volume when the material to be sampled is collected, and configured to release a preloaded substance from the at least one chamber.

8. The ingestible device (10) according to any of the preceding claims, wherein the electronics module (14) further comprises:
at least three chambers (20, 22, 24), wherein the at least one chamber is one of the at least three chambers (20, 22, 24), and
wherein each chamber comprises an inlet that aligns with the opening (32) of the cap (12) to collect the material to be sampled when the cap (12) slides over the electronics module (14).

9. The ingestible device (10) according to claim 8, wherein the electronics module (14) further comprises:
O-rings (40, 42, 44, 46) positioned in a first set of grooves (48) in the electronics capsule, wherein each chamber is sealed by the O-rings and the cap (12) covering the electronics module (14).

10. The ingestible device (10) according to claim 9, wherein the electronics module (14) comprises at least one sensor in the closed off space in between the O-rings (40, 42, 44, 46), and configured to determine one or more parameters of the biological analyte.

11. The ingestible device (10) according to any of the preceding claims, wherein the cap (12) comprises one or more ridges (80) that fit in a second set of grooves (82) in the electronics module (14), and configured to prevent rotation of the cap (12) over the electronics module (14) and prevent the cap (12) from sliding away from the electronics module (14) .

12. The ingestible device (10) according to any of the preceding claims, wherein at least a part of the inner space of the at least one chamber comprises a stabilizing substance, preferably a quencher, especially for preserving the sampled material, wherein the quencher is embedded in the hydrophilic material.

13. The ingestible device (10) according to any of the preceding claims,
wherein the electronics module (14) further comprises a body portion (25) especially providing a hollow inner space being preferably sealed against the environment of the body portion (25).

14. The ingestible device (10) according to claim 13, wherein the body portion (25), especially the hollow inner space of the body portion 925), comprises at least one of:
a trigger unit (57) configured to activate the actuator,
a data recording unit (55) for recording data, preferably environment data and/or localization data, during usage of the ingestible device (10), and
a wireless data transmission unit (53) for wirelessly transmitting data, preferably environment data and/or localization data, more preferably environment data and/or localization data in real time, during usage of the ingestible device (10).

15. The ingestible device (10) according to any of the preceding claims,
wherein the ingestible device (10) has the shape of a pill or a cylinder, and/or
wherein the length of the ingestible device (10) is lower than 31 millimeters after the actuator is activated, and/or wherein the diameter of the ingestible device is lower than 9.9 millimeters.
